**Europäisches Patentamt**

(19) **European Patent Office**

**Office européen des brevets**

(11) Publication number : **0 522 839 A2**

## (12) EUROPEAN PATENT APPLICATION

(21) Application number : **92306264.0**

(22) Date of filing : **08.07.92**

(51) Int. Cl.$^5$ : **C07C 5/367**, C07C 15/44

(30) Priority : **11.07.91 US 728668**

(43) Date of publication of application :
**13.01.93 Bulletin 93/02**

(84) Designated Contracting States :
**AT DE ES FR GB NL**

(71) Applicant : **Union Camp Corporation**
**1600 Valley Road**
**Wayne New Jersey 07470 - 2066 (US)**

(72) Inventor : **Mitchell, Peter W. D.**
**106 Lancaster Road**
**Freehold, New Jersey 07728 (US)**
Inventor : **Sasser, David E.**
**6290 Nancy Drive**
**Jacksonville, Florida 32210 (US)**

(74) Representative : **Hildyard, Edward Martin et al**
**Frank B. Dehn & Co. European Patent**
**Attorneys Imperial House 15-19 Kingsway**
**London WC2B 6UZ (GB)**

(54) **Catalytic dehydrogenation of cyclic dienes.**

(57)  A method is disclosed for the dehydrogenation of cyclic or poly-cyclic olefins, specifically mono- and bi-cyclic terpenes and hydroaromatics, using alkali metals alone or sodium conjoined with amines, which alkali metals and sodium are present in catalytic amounts. The method leads to economical production of dehydrogenated terpene and aromatic products.

EP 0 522 839 A2

This invention relates to dehydrogenation of cyclic dienes and, more specifically, is concerned with dehydrogenation of cyclic terpenes.

## BACKGROUND OF THE INVENTION

Turpentine is a general term used for the volatile oil present in trees, primarily coniferous trees. Chemically, turpentine is predominantly a mixture of unsaturated mono- and bi-cyclic $C_{10}H_{16}$ hydrocarbons. Terpenes, in the strict sense, are volatile hydrocarbons of the empirical formula $C_{10}H_{16}$. The composition of the turpentine is determined by the species of the tree.

Although over 30 compounds have been identified in turpentine, only a few have commercial significance, i.e., they can be separated in high purity for subsequent use. The terpenes will undergo numerous reactions, including hydrogenation, isomerization, polymerization, oxidation, halogenation, esterification, and dehydrogenation.

Terpenes are a renewable resource that may be used to replace the present petroleum-based source of most of industry's hydrocarbons. However, a turpentine or a mixture of terpenes, in and of itself, is not a commercially significant hydrocarbon feedstock. Therefore, a process that will readily convert a terpene or a turpentine feedstock into a valuable or commercially more acceptable compound is highly desirable.

Over the years there have been varying amounts of interest in the availability of para-cymene. Presently, its current relatively high cost has limited its use. At a lower cost, however, para-cymene would be economically viable for use in a variety of new products and would provide additional savings in the manufacture of products in which para-cymene is currently used, such as synthetic musk.

Examples of the production of para-cymene from terpene feedstock are numerous. Such methods have included: disproportionation of mono- or bi-cyclic terpenes or hydroaromatics using palladium or nickel catalysts in both the liquid and vapor phase; dehydrogenation of mono- or bi-cyclic terpenes or hydroaromatics using sulphur or selenium, particularly the production of para-cymene dehydrogenation of alkenylcyclohexene over a catalyst which contains palladium oxide and sulfur and/or selenium or selenium oxide on active carbon (U.S. Patent No. 4,720,603 (Martin et al.)); dehydrogenation of limonene by sodiumbenzyl catalyst; and dehydrogenation of limonene by N-lithioethylenediamine (LEDA) (Reggel et al., J. ORG. CHEM., 23, 1136 (1958)). Other prior art methods include using an oxide of Tb combined with an alkali and/or alkaline earth metal (U.S. Patent No. 4,511,754 (Gaffney)). Each of these methods, however, suffer from substantial drawbacks.

The cost of the catalyst used can make the entire process uneconomical. For example, LEDA works well in the dehydrogenation of terpenes, but the cost of the lithium used is a major drawback. Such cost has mandated the recycling of the catalyst used, thereby complicating the overall process.

Further, if the catalyst chosen is not recycled, problems of disposal of the catalyst used, such as nickel, can arise due to their potential toxic nature.

Alternatively, use of a disproportionation process will result in the production of substantial amounts of unwanted by-product in addition to the desired product. For example, when palladium or nickel is used as a catalyst with limonene, the resulting product mixture contains para-cymene in a ratio of only 2:1 to the also resulting para-menthanes. Nevertheless, this process remains in common use.

Accordingly, there exists a need for a method of easily dehydrogenating mono- or bi-cyclic terpenes that is inexpensive, readily available, that produces relatively pure product, and that does not suffer from the problems of the prior art catalysts.

## SUMMARY OF THE INVENTION

The present invention is directed to a method for the economic dehydrogenation of cyclic olefins, poly-cyclic olefins and mixtures thereof, specifically unsaturated cyclic compounds, and more specifically mono- and bi-cyclic terpenes and hydroaromatics to produce useful products such as para-cymene. Surprisingly, alkali metals alone, including sodium, potassium, and lithium, have been found to be reactive with such unsaturated cyclic compounds. In particular, sodium, alone or in combination with any of a variety of amines, has been found to be reactive with the mono- and bi-cyclic terpenes. Use of alkali metals, preferably sodium, in catalytic amounts achieves substantial dehydrogenation. Further, the use of alkali metals, preferably sodium, renders the process significantly more economical than the use of prior art methods of dehydrogenation.

Accordingly, it is an object of the present invention to provide a method for quickly and economically dehydrogenating mono-and bi-cyclic terpenes and hydroaromatics, thereby producing useful dehydrogenated terpene and aromatic products. Other and further objects and advantages will appear hereinafter.

## DETAILED DESCRIPTION OF THE PREFERRED EMBODIMENTS

It has been shown previously that a catalyst formed in a solution from the reaction of lithium with ethylene diamine, which probably contains LEDA, is capable of catalyzing the dehydrogenation reaction of limonene and its double bound isomers (See, for example, Pines and Stalick, BASE CATALYZED REACTIONS OF HYDROCARBONS AND RELATED COMPOUNDS, Academic Press (1977)). Conversely, the prior art expressly teaches away from the use of N-sodioethylenediamine (NaEDA) as a catalyst for such reactions (id. at 487). The use of NaEDA was specifically discouraged when attempting to dehydrogenate mono- or bi-cyclic terpenes. This was particularly true with respect to unconjugated cyclic dienes. While it was reported that NaEDA would dehydrogenate alpha-phellandrene, a compound having conjugated double bonds, the molar amount of sodium used was three times that of the alpha-phellandrene. It was reported at the same time that NaEDA would not catalyze the conversion of d-limonene, a compound having unconjugated double bonds. (Reggel et al., J. ORG. CHEM., 23, 1136 (1958)).

A further problem with the potential use of NaEDA as compared to the use of LEDA is the difficulty that those of ordinary skill in the art had in making NaEDA. Lithium salts of ethylenediamine can be prepared directly by reacting lithium metal and diamine. It was previously thought, however, that the sodium derivatives could not be made so directly. The normal practice had been to first prepare sodium hydride and then to react the sodium hydride with the diamine. Recently, however, a process was discovered that allowed for a simpler preparation technique utilizing a catalytic proportion of a transition metal compound (U.S. Patent No. 4,521,627 (Mitchell) incorporated herein by reference in its entirety).

Surprisingly, it has been discovered now that sodium conjoined with an amine, wherein the sodium is present in catalytic amounts, can act to catalyze the dehydrogenation of cyclic and poly-cyclic olefins and mixtures thereof, including mono- and bi-cyclic terpenes and hydroaromatics; particularly, unconjugated cyclic dienes. Catalytic amounts as used herein refers to less than stoichiometric amounts on a molar basis, based on the cyclic olefin or poly-cyclic olefin to be dehydrogenated. The method of the present invention can lead to near quantitative yields, employing sodium in catalytic amounts with no need to pre-form a sodium/amine catalytic compound. While this is the preferred path to follow in the present invention, one skilled in the art will appreciate that the sodium/amine catalytic compound could be pre-formed and then added to the olefins to be dehydrogenated and still fall within the intended scope of the present invention. For example, sodamide may be described as "a pre-formed sodium conjoined with amine" compound.

Furthermore, it has also been discovered that alkali metals, including sodium, lithium and potassium, can be used in catalytic amounts without the presence of amines to catalyze the dehydrogenation of cyclic and poly-cyclic olefins and mixtures thereof, including mono- and bi-cyclic terpenes and hydroaromatics. While other alkali metals may be used as catalysts, sodium is the preferred alkali metal catalyst, as it remains a much cheaper material for industrial use than other alkali metal catalysts claimed herein, such as lithium and potassium. This method is clearly the simplest in that it does not involve any additional amine component for reaction and, given the low cost of sodium, there is no need to recover any of the catalyst.

The mono- and bi-cyclic terpenes to which the method of the present invention applies include at least the para-menthadienes. In particular, limonene, alpha-terpinene, alpha-pinene, beta-pinene, gamma-terpinene, terpinolene, isoterpinolene, alpha-phellandrene, beta-phellandrene, as well as delta-3-carene, can be used advantageously with the method of the present invention. Additionally, hydroaromatic compounds such as 9,10-dihydroanthracene, 1,2-dihydronaphthalene and cyclohexadiene may be dehydrogenated using the methods of this invention. These precursors can be dehydrogenated individually or as feedstock mixtures of various cyclic or poly-cyclic olefins. The dehydrogenation of the present inventive methods acts to aromatize the mono- or bi-cyclic terpene precursors with the concomitant evolution of hydrogen gas. One specifically preferred product is para-cymene, although naphthalene, anthracene, meta-cymene, and other dehydrogenated mono- and bi-cyclic terpenes can be readily produced.

As the examples discussed below illustrate, it has been found that the dehydrogenation of mono- or bi-cyclic terpenes and hydroaromatics can be catalyzed with sodium in any of several fashions. The sodium can be simply added directly to the terpene or hydroaromatic precursor. Alternatively, it can be added to the precursor in combination with an amine. Such amines can be primary, secondary, or tertiary. Specific examples of such amines include ethylenediamine (EDA) and diethylenetriamine (DETA). Other available sodium/amine combinations include sodamide (NaNH$_2$) and the addition of sodium followed by introduction of dry ammonia, preferably by subsurface addition of the dry ammonia into the reaction mixture.

The method of the present invention surprisingly eliminates the need for a transition metal compound catalyst to stimulate the formation of the sodium/amine compound as had been required previously. Nevertheless, it is clear that such transition metal compound catalysts may still be used without detrimental effect.

The rate at which the dehydrogenation reaction proceeds depends on a variety of factors, including the

3

temperature, the pressure, the amount of each component present, and whether or not the process is operated as a batch process or as a continuous process. In the batch processes of the examples discussed below, typical reaction times range from 2 to 10 hours. The present invention, however, can be readily modified to be used with a continuous process.

The alkali metals may be added in any of a variety of forms. Solid lumps can be used, although this severely limits the available surface area and, as a result, tends to significantly slow the reaction rate. Since the melting points of sodium and potassium are less than 100°C, it is also possible to introduce those alkali metals as a molten liquid. It is most preferable, however, to utilize dispersions of alkali metals in mineral oil. This form is preferred in that it exposes the maximum surface area of the metal while providing a convenient way to add the catalyst to the precursor feedstock.

The methods of the present invention are conducted at temperatures and pressures effective to induce the dehydrogenation of the cyclic or poly-cyclic olefin. Preferred temperatures of reaction of the method of the present invention range from about 50° to about 350°C. More preferably, the temperature is maintained between 100°C and 190°C (185°C being the boiling point of para-cymene). The pressures used in the present invention may be atmospheric or sub-atmospheric. Preferred sub-atmospheric pressures range from about 100-200 mmHg. One preferred method of dehydrogenating is to place the components under reflux conditions.

The preferred catalytic amount of sodium used is 0.1-2.0% by weight of cyclic or poly-cyclic olefin being dehydrogenated. It is not believed, however, that any specific harm results from the use of excess sodium other than potential increased costs. Thus, while a prime advantage of the present process is the ability to cut cost by using catalytic amounts of sodium, the process of the present invention contemplates using greater amounts of sodium as well.

To optimize reaction rates, sodium conjoined with an amine is the preferred catalytic compound. One preferred embodiment of the present invention is to utilize pure sodium, added as a dispersion, followed by the introduction of dry ammonia by subsurface addition into the sodium/feedstock reaction mixture. As the feedstock reaction mixture approaches complete conversion to the corresponding aromatic product, the product is easier to isolate or purify for later use.

It has been found that the use of sodium conjoined with EDA or DETA does not require as high a temperature to drive the reaction to complete conversion as do the other embodiments discussed herein. Therefore, for commercial scale-up, the use of one of these amine compounds conjoined with the sodium is more preferred. Because of the economics involved, if sodium is conjoined with EDA, it is preferable, but not required, to recover the EDA.

A significant advantage of the method of the present invention is the low cost of the catalyst. In addition to the relatively low cost of sodium, the ability to use sodium and other alkali metals in a catalytic amount further decreases the overall cost of the process.

A further advantage of the present invention is the near quantitative yield of desired product. While disproportionation reactions of the prior art incur some hydrogen transfer and, thus, end up with large quantities of unwanted by-product, the present method avoids hydrogen transfer, the hydrogen evolving as a gas for easy removal.

The following examples describe the manner and process of making and using the method of the present invention and set forth the preferred embodiments contemplated by the inventors of carrying out their invention. They are not to be construed, however, as limiting the scope of the present invention.

Examples 1-6 below demonstrate the dehydrogenation of mono- and bi-cyclic terpenes to form para-cymene, utilizing catalytic amounts of conjoined sodium and amine. Example 7 demonstrates the dehydrogenation of a hydroaromatic 9,10-dihydroanthracene to form anthracene, also utilizing catalytic amounts of conjoined sodium and amine. Examples 8-11 demonstrate the conversion of a cyclic terpene to para-cymene using catalytic amounts of alkali metals in the absence of any amine. Examples 12-14 demonstrate the dehydrogenation of a feedstock mixture composition, which feedstock comprises primarily mono- and bi-cyclic terpenes, utilizing catalytic amounts of conjoined sodium and amine.

Table 1 is a summary for Examples 12-14 of the weight percent of each component in the initial feedstock and in the reacted final product. The results found in Table 1 and in Example 4 are weight percent values as determined by gas chromatography, utilizing an internal standard method. The results found in all other examples are area percent values as determined by gas chromatography with no internal standard.

## EXAMPLE 1

A mixture of ethylenediamine (EDA, 7.8g), anhydrous ferric chloride (0.04g) and sodium (0.82g; 36 mmol) was warmed to about 50°C. After sodium dissolution had started, as evidenced by hydrogen evolution, limonene (5g) was added and the mixture heated to about 100°C. When the vigorous reaction had subsided, an additional quantity of limonene (5g) was added. Three more aliquots of limonene (5g each) were added after the previous aliquot's reaction had subsided.

The mixture was diluted with water and the precipitated oil was washed with water. The oil weighed 23g and contained 92.8% (157 mmol) of para-cymene and 2.0% limonene.

EXAMPLE 2

A mixture of sodamide (1.38g; 35 mmol) and EDA (7.5g) was stirred and heated to 100°C to give a blue solution. Then, limonene (72g) was added over a period of 80 minutes. Gas evolution was vigorous at first, but had slowed down by the time that the addition was complete and ceased shortly thereafter.

A sample of the crude washed product was analyzed and found to contain 49.5% para-cymene and 42.1% limonene.

EXAMPLE 3

A slurry of sodamide (1g; 25.6 mmol) in para-cymene (10g) was stirred and heated to reflux. Then, alpha-terpinene (25g) was added over a 2 hour period and refluxing was continued for an additional 3 hours. The crude product contained 95.6 wt% para-cymene and no alpha-terpinene.

EXAMPLE 4

A slurry of sodamide (1g; 25.6 mmol) in para-cymene (10g) was stirred and heated to reflux. Then, alpha-terpinene (60g) was added over a 1-hour period. Sampling showed that the reaction was complete after 7 hours of reflux. From the reaction mixture, para-cymene (48.5g) of 98.5% purity was removed by flash distillation at 100 mmHg pressure. To the remaining heel of catalyst-containing para-cymene additional alpha-terpinene (60g) was added and refluxing was continued. To increase the rate of reaction, additional sodamide (0.1g) was added. After a total of 17 hours of reflux the mixture was found to contain 42.1% alpha-terpinene and 53.2% para-cymene.

EXAMPLE 5

A mixture of alpha-terpinene (60g) and sodium dispersion (2.5g of 40 wt% sodium in mineral oil; 43.5 mmol) was heated to reflux. After 1 hour of reflux, the mixture contained 7.8% para-cymene. At this point, a slow stream of dry ammonia was introduced into the subsurface of the refluxing mixture. In 1 hour, the para-cymene content had risen to 50.1%. In a further 1 hour, reaction was complete. Additional alpha-terpinene (60g) was added to the batch and refluxing with ammonia addition was continued. After 2.5 hours, the product contained 96.1 wt% para-cymene.

EXAMPLE 6

The method of example 5 was repeated except that delta-3-carene (a bicyclic mono-olefin) was used instead of alpha-terpinene. After 20 hours of reflux in the presence of ammonia, the reaction mixture contained 8.4% meta-cymene and 20% para-cymene with 53% delta-3-carene remaining.

EXAMPLE 7

A mixture of 9,10-dihydroanthracene (10g), para-cymene (10g), and sodamide (1g) was refluxed at 185°C for 24 hours. An analyzed sample, excluding solvent, contained 29.4% 9,10-dihydroanthracene and 69.9% anthracene, the latter being identified by its GC retention time.

EXAMPLE 8

A mixture of para-cymene (10g), used as a solvent, and sodium dispersion (2.5g of 40 wt% sodium in mineral oil; 43.5 mmol) was heated to reflux and alpha-terpinene (60g) was added over a period of 1.5 hours. Para-cymene/alpha-terpinene analyses showed 71%/23% (23 hrs), 86.9%/5% (31 hrs), and 92.5%/0.5% (36 hrs).

EXAMPLE 9

A mixture of limonene (27.2g, 0.2 moles) and potassium hydride dispersion (16.0g of 25 wt% potassium in mineral oil, 0.1 moles) was stirred and heated to reflux. After 64 hours at reflux, analysis by GC showed 11.9% limonene and 57.1% para-cymene (114 mmol) as well as 28.1% of an unidentified earlier eluting component.

EXAMPLE 10

A mixture of alpha-terpinene (27.2g, 0.2 mole) and lithium dispersion (2.8g of 25 wt% lithium containing 0.5% sodium, 0.1 mole) was heated to reflux. After a total of 57 hours at reflux conversion of alpha-terpinene was complete, yielding a product containing 92.2% para-cymene. This represents 2 turnovers of the lithium catalyst.

From a practical standpoint, most products obtained using the process of the present invention will be produced from the dehydrogenation of a mixture of terpenes. Such mixtures typically contain 15-20 different species. Several examples were run using typical industrial mixture samples as feedstock. The results of the following examples, Examples 11-13, are summarized in Table 1.

EXAMPLE 11

A mixture of limonene (20.2g, 0.148 moles) and sodium dispersions (2.5g of 40 wt % dispersion in mineral spirits, 0.044 moles) was heated to reflux. After 1 hour at reflux 60.1g (0.442 moles) additional limonene was added and the reaction was continued. After 48 hours total at reflux, the reaction was quenched with isopropanol and transferred with ether. After washing with water and saturated brine solutions, the ether solution was dried ($K_2CO_3$) and evaporated to yield 77.5g of crude product containing 6.2% para-cymene, 76.6% limonene, and 15.9% isoterpinolene using wt % internal standard method of analysis.

EXAMPLE 12

Mixed feedstock (2500g), as described in Table 1, was charged to a flask equipped with a 2.5 theoretical plate column/Dean Stark trap. At 100 mmHg pressure, reflux was established and water was removed. At 105°C, EDA (500g) and sodium (25g) were charged to the flask. After an induction period, hydrogen evolution began and the temperature was decreased to moderate the reaction rate. After 3.3 hours (from addition of sodium), a product containing 86.5% para-cymene was obtained.

EXAMPLE 13

Mixed feedstock (2500g), as described in Table 1, EDA (500g), and sodium (25g) were charged in a similar manner to a flask equipped with a Dean Stark trap. After an induction period, hydrogen evolution began and the EDA was refluxed back to the reactor. After 4.4 hours, the product containing 92% para-cymene was obtained.

It was desired to recover the EDA prior to quenching the sodium in order to obtain the driest EDA for recycle so long as this did not result in significant losses of EDA. Distillation of the unquenched reaction mixture should also promote complete conversion of the mixture of para-menthadiene, which will facilitate the final fractionation of the para-cymene. Thus, the following approach was used: distillation of the unquenched reaction mixture; 200 mmHg overhead pressure; 5:1 reflux ratio; and use of a 10 theoretical plate column. Results indicated that the EDA recoveries were much improved by this approach and that the level of EDA required could be substantially reduced without incurring a further increase in loss of EDA.

EXAMPLE 14

Mixed feedstock (500g), as described in Table 1, was charged to a dry flask along with diethylenetriamine (DETA, 50g) and ferric nitrate (0.1g). The dry flask had been purged with nitrogen heated to 105-110°C. Sodium (13g) was added and the mixture was stirred. Additional mixed feedstock (500g) was added dropwise once hydrogen evolution slowed. Additional sodium (4g) was added after 8 hours, followed by additional DETA (150g) at 12.4 hours, at which time no reaction was observed. Finally, additional DETA (20g) was added after 25 hours. After 29.5 hours, a product containing 88.8% para-cymene was obtained. It was apparent that the initial loading of 5 weight percent DETA failed to initiate the reaction. Thus, the additional DETA (a total of 20 weight percent) was added to allow the reaction to initiate and go to completion.

## TABLE 1

### INITIAL FEEDSTOCK/FINAL PRODUCT COMPOSITION

| Mixed Feedstock Components | Example 12 | | Example 13 | | Example 14 | |
|---|---|---|---|---|---|---|
| | % in Init. Feed | % in Final Prod. | % in Init. Feed | % in Final Prod. | % in Init. Feed | % in Final Prod. |
| Tricyclene | 1.3 | 0.9 | 1.1 | 0.2 | 0.7 | 0.7 |
| Camphene | 5.0 | 4.7 | 4.5 | 2.3 | 3.7 | 4.0 |
| Myrcene/ Alpha-Phellandrene | 1.0 | 0.0 | 1.1 | 0.0 | 1.1 | 0.0 |
| Carvomenthenes | 0.0 | 1.3 | 0.0 | 0.9 | 0.0 | 1.0 |
| Alpha-Terpinene/ 1,4-Cineole | 39.6 | 0.0 | 39.7 | 0.0 | 42.3 | 0.1 |
| Limonene | 0.7 | 0.0 | 0.7 | 0.0 | 0.8 | 0.2 |
| Beta-Phellandrene | 0.5 | 0.0 | 0.5 | 0.0 | 0.5 | 0.0 |
| 1,8-Cineole | 0.2 | 0.2 | 0.2 | 0.1 | 0.2 | 0.2 |
| Gamma-Terpinene | 17.5 | 0.0 | 17.2 | 0.0 | 19.3 | 0.1 |
| 3,8-p-Menthadiene | 2.7 | 0.0 | 2.8 | 0.0 | 2.7 | 0.0 |
| Para-Cymene | 1.0 | 86.5 | 1.6 | 92.0 | 1.0 | 88.8 |
| Terpinolene | 2.3 | 0.0 | 2.1 | 0.0 | 2.7 | 0.0 |
| Isoterpinolene | 21.0 | 0.0 | 21.8 | 0.0 | 20.1 | 0.5 |
| 3,8-m-Menthadiene | 0.1 | 0.0 | 0.1 | 0.0 | 0.1 | 0.0 |
| Alcohols | 1.8 | 2.6 | 1.7 | 1.9 | 1.2 | 1.6 |
| High Boilers | 0.9 | 0.8 | 1.4 | 1.1 | 0.1 | 0.0 |
| Others | 4.4 | 3.0 | 3.6 | 1.4 | 3.5 | 2.6 |

It should be clear that strict adherence to many of the particular parameters chosen for the examples above, e.g., amounts of reactants used, temperatures, time periods, etc., is not critical to achieve the desired results. In addition, particular variations of the synthesis steps would also be apparent to those of ordinary skill in the relevant field.

Thus, a method of easily dehydrogenating mono- and bi-cyclic terpenes and hydroaromatics using catalytic amounts of alkali metals, preferably sodium, and sodium conjoined with an amine is disclosed. While embodiments and applications of this invention have been shown and described, it would be apparent to those skilled in the art that many more modifications are possible without departing from the inventive concept herein. The invention, therefore, is not to be restricted except in the spirit of the appended claims.

## Claims

1. A method of dehydrogenating cyclic olefins, poly-cyclic olefins and mixtures thereof, the method comprising: contacting the cyclic and/or poly-cyclic olefins to be dehydrogenated with a catalytic amount of an alkali metal at temperatures and pressures effective to induce the dehydrogenation of the cyclic and/or poly-cyclic olefins.

2. The method of claim 1 wherein the alkali metal is selected from the group consisting of sodium, potassium and lithium.

3.   A method for dehydrogenating cyclic olefins, poly-cyclic olefins and mixtures thereof, the method comprising: contacting the cyclic and/or poly-cyclic olefins to be dehydrogenated with sodium conjoined with an amine at temperatures and pressures effective to induce the dehydrogenation of the cyclic and/or poly-cyclic olefins, wherein the sodium is used in catalytic amounts.

4.   A method as claimed in claim 3 effected under conditions of reflux.

5.   The method of claim 3 or claim 4 wherein the amine is selected from diamines, triamines, and tetramines.

6.   The method of claim 3 or claim 4 wherein the amine is selected from ethylenediamine, diethylenetriamine and ammonia.

7.   The method of claim 3 or claim 4 wherein the sodium conjoined with an amine comprises sodamide.

8.   The method of any one of claims 3 to 7 further comprising the addition of a transition metal compound.

9.   The method of claim 8 wherein the transition metal compound is ferric nitrate or ferric chloride.

10.   The method of any one of claims 4 to 9 wherein the amine level is from about 10% to about 30% by weight of the olefins to be dehydrogenated.

11.   The method of any one of the preceding claims wherein the cyclic or poly-cyclic olefins are selected from mono-cyclic terpenes, bi-cyclic terpenes and hydroaromatics.

12.   The method of claim 11 wherein the cyclic terpenes are selected from limonene, terpinolene, isoterpinolene, alpha-phellandrene, beta-phellandrene, 3,8-para-menthadiene, 2,8-para-menthadiene, 3,8-meta-menthadiene, alpha-terpinene, gamma-terpinene, alpha-pinene, beta-pinene and delta-3-carene.

13.   The method of claim 11 wherein the hydroaromatics are selected from the group consisting of cyclohexadiene, 9,10-dihydroanthracene and 1,2-dihydronaphthalene.

14.   The method of any one of the preceding claims wherein the temperature is between about 50°C and about 350°C.

15.   The method of claim 14 wherein the temperature is between about 100°C and about 190°C.

16.   The method of claim 14 or claim 15 wherein the pressure is about one atmosphere or less than one atmosphere.

17.   The method of claim 6 wherein the pressure is about 100 mmHg and the temperature is from about 100°C to about 110°C.